# EUROPEAN PATENT SPECIFICATION

(11) **EP 0 871 699 B1**
(45) Date of publication and mention of the grant of the patent: **22.01.2003**
(21) Application number: 96935053.7
(22) Date of filing: 18.10.1996
(51) Int. Cl.: C12M 1/36, C12G 1/02, C12C 11/00, G01N 33/00

(54) **METHOD OF MAKING AN ALCOHOLIC BEVERAGE BY FERMENTATION, AND FERMENTATION TEST KIT FOR USE IN THE METHOD**
VERFAHREN ZUR HERSTELLUNG EINES ALKOHOLISCHES GETRÄNK DURCH FERMENTATION UND FERMENTATIONSTESTSATZ ZUR DURCHFÜHRUNG DES VERFAHRENS
PROCEDE DE PRODUCTION D'UNE BOISSON ALCOOLIQUE PAR FERMENTATION ET TROUSSE D'ESSAI ASSOCIEE

(30) Priority: 18.10.1995 ZA 9508792; 11.07.1996 ZA 9605899
(43) Date of publication of application: 21.10.1998
(73) Proprietor: Rymco (Pty) Ltd, Industria, Johannesburg 2093 (ZA)
(72) Inventor: VOS, Petrus Johannes Andreas, Stellenbosch 7600 (ZA)
(74) Representative: Sturt, Clifford Mark
(86) International application number: GB9602578
(87) International publication number: WO97014781

(56) References cited:
- EP-A- 0 469 998
- GB-A- 1 210 295
- US-A- 5 356 458
- AMERICAN JOURNAL OF ENOLOGY AND VITICULTURE, vol. 45, no. 1, 1994, USA, pages 107-112, XP000646024 GIUDICI, P. & KUNKEE, R.E.: "The effect of nitrogen deficiency and sulfur-containing amino acids on the reduction of sulfate to hydrogen sulfide by wine yeasts."
- DATABASE WPI Section Ch, Week 7502 Derwent Publications Ltd., London, GB; Class E36, AN 75-02934W XP002027547 & JP 49 046 276 B (DENKI KAGAKU KEIKI CO) , 9 December 1974
- PATENT ABSTRACTS OF JAPAN vol. 009, no. 246 (P-393), 3 October 1985 & JP 60 098336 A (GENSHI NENRIYOU KOGYO KK), 1 June 1985,
- AMERICAN JOURNAL OF ENOLOGY AND VITICULTURE, vol. 46, no. 2, 1995, USA, pages 269-273, XP000626026 JIRANEK, V. ET AL.: "Validation of bismuth-containing indicator media for predicting h2s-producing potential of Saccharomyces cerevisiae wine yeasts under enological conditions."

## Description

This invention relates to a method of making an alcoholic beverage by fermentation,

During the fermentation of, for example, grape musts, the wine yeasts responsible for fermentation require the presence of sugar and free amino nitrogens (also referred to as "FAN") as essential nutrients for fermentation. When making wine from musts that have a sub-optimal FAN content, lagging (i.e. slow, retarded, or stuck) fermentations accompanied by the formation of off-flavours are often encountered, which result in poor wine quality. It is known for deficiencies in FAN levels to be rectified by the addition of diammonium phosphate (also referred to as "DAP"). DAP is a source of assimilable nitrogen and, when added in sufficient quantities, effectively restores any deficiency in the nutrient requirement of yeasts during fermentation.

Traditional techniques (such as the ninhydrin methodology) of controlling the FAN levels in grape musts involve a determination of assimilable nitrogen levels in the musts, and adjustments to make up for any deficiencies, prior to fermentation. The ninhydrin determination of assimilable nitrogen levels requires specialized knowledge and the use of expensive apparatus in controlled laboratory conditions, which are normally well beyond the ability and means of the average wine maker.

Guidici, P. and Kunkee, R.E. ("The effect of nitrogen deficiency and sulfur-containing amino acids on the reduction of sulfate to hydrogen sulfide by wine yeasts", Am. J. Enol. Vitic., Vol.45, no.1, 1994, pages 107-112) disclose that nitrogen deficiency in grape musts leads to the production of H₂S, and that the presence of H₂S can be detected by lead acetate impregnated strips. They stress that the experimental methods described are qualitative only, and that for the better control of H₂S formation under practical wine making conditions a quantitative determination of nitrogen deficiency is required.

RÖMPP CHEMIE LEXICON, Georg Thieme Verlag, Stuttgart-New York 9th Ed., 1992, page 4075 discloses the use of zinc acetate solutions generally, to obtain a quantitative indication of H₂S.

It is an object of the present invention to provide a method of making an alcoholic beverage, and more particularly wine, which enables the FAN content of the fermenting liquor to be adjusted to appropriate levels in a manner that is inexpensive and can readily be implemented by the average maker of such beverage, without the need for specialized knowledge or expensive apparatus and access to laboratory facilities.

According to the invention there is provided a method of making an alcoholic beverage by fermentation of a fermentable liquor, in which a source of assimilable nitrogen is added to the liquor to supplement the assimilable nitrogen content of the liquor, wherein gas evolving from the liquor during fermentation is monitored for the presence therein of H₂S, and wherein said source of assimilable nitrogen is added to the liquor during the course of fermentation, in response to the detection of H₂S in said gas.

The method may comprise the steps of (a) monitoring said gas for the presence therein of H₂S, for a predetermined period, (b) thereupon adding a portion of said source of assimilable nitrogen to the liquor in response to the detection of H₂S in said gas during that period, and (c) repeating the steps (a) and (b).

The steps (a) and (b) may be repeated continuously, from the beginning to the end of the fermentation.

Said gas may be monitored continuously during each said period.

The steps (a) and (b) may be repeated at least once a day.

Where the fermentation is a relatively cold or slow fermentation, the steps (a) and (b) may be repeated once a day. Where the fermentation is a relatively warm or vigorous fermentation, the steps (a) and (b) may be repeated at least once every 8 hours.

Said gas may be monitored for the presence therein of H₂S by exposing an indicator strip which is selectively responsive to the presence of H₂S, to the gas.

The indicator strip may be a zinc acetate impregnated indicator strip, and may, after exposure to said gas, be reacted with a first reagent comprising a solution of 4-amino-N,N-dimethylaniline sulphate (or its hydrochloride), to which a second reagent comprising a solution of ferric chloride is added, whereby the development of a blue colour in the admixture of said reagents establishes the presence of H₂S in said gas.

The invention will now be described in more detail, by way of example, with reference to the accompanying drawings.

In the drawings:
Figure 1 illustrates the mechanism of H₂S development during fermentation;
Figure 2 illustrates a method of making wine in accordance with the invention; and
Figure 3 illustrates a fermentation test kit in accordance with the invention.

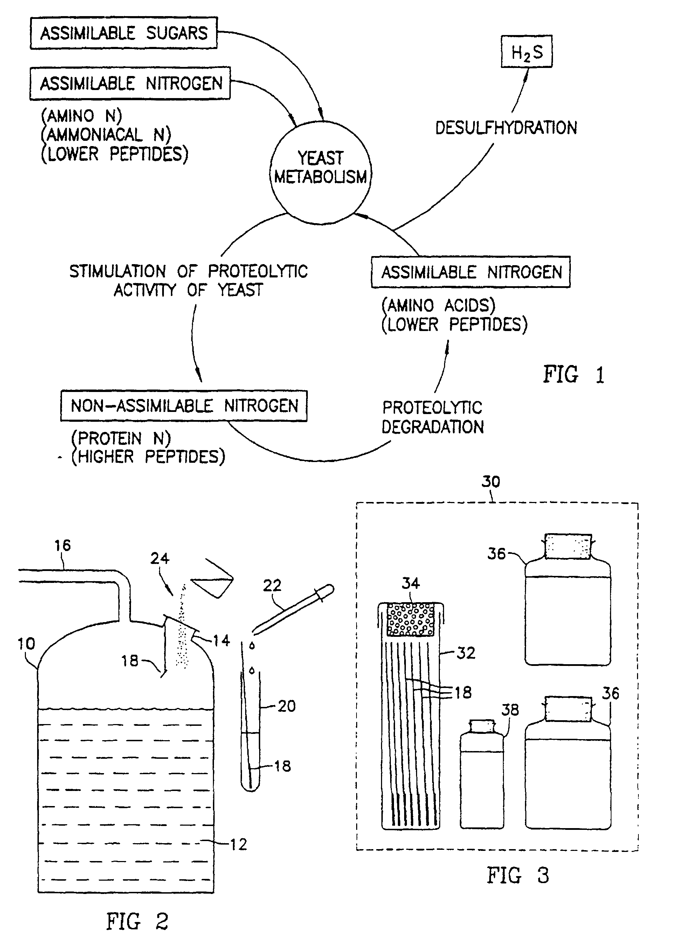

As illustrated in Figure 1, yeast metabolism requires, as essential nutrients in the fermenting liquor, assimilable sugars and assimilable nitrogens or FAN (amino acids, lower peptides, and ammoniacal nitrogen).

Under conditions of sub-optimal FAN content, the proteolytic activity of the yeast is stimulated. This causes the yeast to act on sources of non-assimilable nitrogen (proteins and higher peptides in musts and other fermenting liquors), to form the required assimilable nitrogen through proteolytic degradation. Through this activity, the sulphur derivatives of protein (e.g. cysteine) are desulphurised and liberated as H₂S. It follows that there is an inverse relationship between the assimilable nitrogen content of the fermenting liquor and the development of H₂S during fermentation of the liquor. The presence of H₂S in the gas evolving from the fermenting liquor is a direct result of assimilable nitrogen deficiency experienced by the yeast, and is thus directly related to the nitrogen demand of the particular yeast when the assimilable nitrogen content of the liquor is at a sub-optimal level.

Referring now to Figure 2, reference numeral 10 indicates a fermentation vessel in which grape musts 12 are fermented. The vessel has a manhole 14, and a duct 16 via which gases evolving from the fermenting liquor can leave the vessel.

In implementing the present invention the manhole 14 is opened periodically, an unexposed indicator strip 18 suspended in the space above the fermenting liquor 12, and the manhole closed again. The indicator strip is thus continuously exposed to the stream of gas evolving from the fermenting liquor. Each time a new, unexposed indicator strip is inserted in the vessel, the previously inserted, now exposed, indicator strip is removed from the vessel. Each indicator strip should preferably be exposed to the gas evolving from the fermenting liquor for a continuous period of up to 24 hours. In the case of slower or colder fermentations exposure for a continuous period of 24 hours, and replacement of the strip once a day, is recommended. In the case of more vigorous or warmer fermentations exposure for a continuous period of up to 8 hours, and replacement of the strip at least three times a day, is recommended.

The indicator strips 18 are plastic strips which have a piece of filter paper at their tips, the strips having been sensitised by impregnation of the filter paper with a 6% solution of zinc acetate (Zn(CH₃COO)₂.2H₂O) and subsequent drying.

When inserting a new, unexposed indicator strip in the vessel the previously, now exposed, indicator strip is removed from the vessel and developed. This is done by inserting the indicator strip into a test tube 20 which contains 5mℓ of a first reagent, to which 5 drops of a second reagent are added by means of a dropper 22. The first reagent comprises a 0,02% solution of 4-amino-N,N-dimethylaniline sulphate (or its hydrochloride) in 0,1N HCl, and the second reagent comprises a 2,7% solution of ferric chloride (FeCl₃.6H₂O) in 0,1N HCl. The contents of the test tube are shaken vigorously and then left for 30 minutes for maximum colour development. Development of a blue colour (methylene blue) is a positive indication of the presence of sulphide and thus a nitrogen deficiency of the particular fermenting liquor. The colour intensity is indicative of the degree of nitrogen deficiency (and hence the nitrogen demand of the particular yeast strain). If the test indicates a nitrogen deficiency, a predetermined quantity of DAP or other suitable source of assimilable nitrogen is added to the liquor 12, as indicated at 24. The amount of DAP added may vary according to the colour intensity obtained when the indicator strip 18 is developed. For example, a colour chart may be provided to enable the wine maker to differentiate between light blue, intermediate blue, and dark blue, and 10g/hℓ, 20g/hℓ, or 30g/hℓ of DAP added depending on whether light, intermediate, or dark blue is indicated. Normally, no more than 30g/hℓ of DAP should be added at a time, as this may result in over-dosing.

The more frequent the above procedure is repeated, the less will the FAN content of the fermenting liquor deviate from the optimal value. It has been found that, with colder, slower fermentation processes such as the ones required for making white wine, a frequency of once per day (exposure of the strips for a continuous period of 24 hours) gives good results. With warmer, faster fermentation processes such as the ones required for making red wine, however, a frequency of 3 times per day (exposure of the strips for a continuous period of 8 hours) may be required.

Table 1 at the end of the specification indicates the results of tests carried out on six of the most popular yeast strains used in South Africa. From this it is evident that, according to the traditional method, the nitrogen-deficient control samples required the addition of 45g/hℓ DAP prior to fermentation. When compared to control samples to which no DAP was added, the addition of DAP according to the traditional method resulted in an average decrease of 58,8% in the formation of hydrogen sulphide (i.e. the nitrogen demand of the yeast strains). In contrast, the new method required an average addition of only 33g/hℓ DAP and resulted in an average decrease of 81,7% in the formation of hydrogen sulphide.

It is evident from column 2 in Table 1 that there are significant differences in the production of hydrogen sulphide among individual strains (i.e. differences in their nitrogen demand). In the case of traditional methods these differences are not taken into consideration. In the new method, however, the nitrogen demand of each particular yeast strain is catered for as fermentation progresses. The new method therefore recognises the most important variables that influence the metabolic functions of a particular fermentation.

The materials for enabling the wine maker to implement the method may be provided in the form of a fermentation test kit, such as the one illustrated at 30 in Figure 3. The kit 30 comprises a tube 32 containing a supply of sensitised indicator strips 18, the tube being closed in an air-tight manner by means of a cap which includes a desiccator 34, one or more containers 36 containing a supply of the first reagent, and a container 38 for containing a supply of the second reagent. The kit 30 may further include a test tube and a dropper such as the ones illustrated in Figure 2.

By implementing the technique as outlined above in the making of wine, the wine maker can detect sub-optimal conditions as soon as they occur, immediately take remedial action, and within a short period thereafter detect the effect of the remedial action by exposing a new indicator strip to the gas evolving from the fermenting liquor for the recommended period and repeating the test. He can moreover do so in a relatively simple and inexpensive manner, without the need for specialized knowledge or access to expensive laboratory facilities, and without having any knowledge of the actual FAN content of the musts. Thus, the technique enables him, in response to detecting the presence of H₂S, to add small quantities of DAP at a time, to monitor the effect of such additions, and to continue the procedure until just enough DAP has been added for signs of the presence of H₂S to disappear. As a consequence, over-dosing with DAP is avoided, and there is no need to determine the FAN content prior to fermentation.

A large number of genetically different yeast strains for fermentation of grape musts are commercially available. The demand for assimilable nitrogen or different strains varies significantly The method of the present invention recognises and accommodates the individual demand of each particular yeast strain. This is a very important additional advantage which cannot be achieved when adjustments for nitrogen deficient musts are made prior to fermentation.

A further advantage of the technique is that the cooling requirements of the fermenting musts are significantly reduced. In existing techniques the quantity of DAP that has to be added for optimal fermentation (as determined by laboratory analysis) is normally all added at the beginning of the fermentation process. The abundance, as a result, of FAN in the fermenting musts at the early stages of fermentation leads to a highly increased fermentation activity which, in turn, leads to excessive generation of heat. This heat has to be removed from the fermenting musts by cooling. With the technique of the present invention there is only a moderate increase in fermentation activity, thus limiting the generation of heat.

The method of the present invention applies to all fermented alcoholic beverages.

**TABLE 1**

| H₂S FORMATION AT 15°C RELATIVE ABSORPTION UNITS | | | | | |
|---|---|---|---|---|---|
| 1 | 2 | 3 | 3A | 4 | 4A |
| | | TRADITIONAL | | NEW METHOD | |
| STRAIN | CONTROL | FAN | DAP | FAN | DAP |
| N 96 | 4,099 | 1,167 | 45 | 1,915 | 20 |
| VIN 13 | 23,160 | 2,627 | 45 | 2,373 | 15 |
| VIN 7 | 44,141 | 12,031 | 45 | 4,229 | 5 |
| WE 372 | 49,260 | 19,069 | 45 | 13,260 | 35 |
| WE 14 | 103,425 | 75,641 | 45 | 38,654 | 60 |
| 228 | 203,764 | 78,197 | 45 | 22,740 | 65 |
| AVERAGE | 75,808 | 31,455 | 45 | 13,861 | 33 |
| %REDUCTION | | -58,5 | | -81,7 | -26 |
| Column 1: Yeast strain Column 2: Control (H₂S values). Column 3: Traditional method of determining the nitrogen demand of yeast strains prior to fermentation (H₂S values). Column 3A: Diammonium phosphate additions in respect of traditional method in g/hℓ. Column 4: New method of determining the nitrogen demand of yeast strains during fermentation (H₂S values). Column 4A: Diammonium phosphate additions in respect of new method in g/hℓ. | | | | | |

## Claims

1. A method of making an alcoholic beverage by fermentation of a fermentable liquor, in which a source of assimilable nitrogen is added to the liquor to supplement the assimilable nitrogen content of the liquor, **characterized in that** gas evolving from the liquor during fermentation is monitored for the presence therein of H₂S, and **in that** said source of assimilable nitrogen is added to the liquor during the course of fermentation, in response to the detection of H2S in said gas.

2. A method as claimed in claim 1, **characterized in that** it comprises the steps of (a) monitoring said gas for the presence therein of H₂S, for a predetermined period, (b) thereupon adding a portion of said source of assimilable nitrogen to the liquor in response to the detection of H₂S in said gas during that period, and (c) repeating the steps (a) and (b).

3. A method as claimed in claim 2, **characterized in that** the steps (a) and (b) are repeated continuously, from the beginning to the end of the fermentation.

4. A method as claimed in claim 2 or claim 3, **characterised in that** said gas is monitored continuously during each said period.

5. A method as claimed in any one of claims 2 to 4, **characterized in that** the steps (a) and (b) are repeated at least once a day.

6. A method as claimed in claim 5, **characterized in that** the steps (a) and (b) are repeated once a day in the case of a relatively cold or slow fermentation.

7. A method as claimed in claim 5, **characterized in that** the steps (a) and (b) are repeated at least 5 once every 8 hours in the case of a relatively warm or vigorous fermentation.

8. A method as claimed in any one of the preceding claims, **characterized in that** said gas is monitored for the presence therein of H₂S by exposing an indicator strip which is selectively responsive to the presence of H₂S, to the gas.

9. A method as claimed in claim 8, **characterized in that** the indicator strip is a zinc acetate impregnated indicator strip, and **in that**, after exposure to said gas, the indicator strip is reacted with a first reagent comprising a solution of 4-amino-N,N-dimethylaniline sulphate (or its hydrochloride), to which a second reagent comprising a solution of ferric chloride is added, whereby the development of a blue colour in the admixture of reagents establishes the presence of H₂S in said gas.

## Patentansprüche

1. Verfahren zur Herstellung eines alkoholischen Getränks durch Fermentierung einer fermentierbaren Flüssigkeit, wobei der Flüssigkeit eine Quelle von assimilierbarem Stickstoff zugesetzt wird, um die Menge an assimilierbarem Stickstoff in der Flüssigkeit zu ergänzen, **dadurch gekennzeichnet, dass** Gas, das sich während der Fermentierung aus der Flüssigkeit entwickelt, auf das Vorhandensein von H₂S darin überwacht wird, und dass die Quelle an assimilierbarem Stickstoff der Flüssigkeit während des Ablaufs der Fermentierung als Reaktion auf die Erfassung von H₂S in dem Gas zugesetzt wird,

2. Verfahren nach Anspruch 1, **dadurch gekennzeichnet, dass** es die Schritte aufweist von (a) Überwachen des Gases auf das Vorhandensein von H₂S darin für eine bestimmte Zeitspanne, (b) anschließendes Zusetzen eines Teils der Quelle von assimilierbarem Stickstoff zu der Flüssigkeit als Reaktion auf die Erfassung von H₂S in dem Gas während der Zeitspanne, und (c) Wiederholen der Schritte (a) und (b).

3. Verfahren nach Anspruch 2, **dadurch gekennzeichnet, dass** die Schritte (a) und (b) von dem Anfang bis zur Beendigung der Fermentierung ununterbrochen wiederholt werden,.

4. Verfahren nach Anspruch 2 oder Anspruch 3, **dadurch gekennzeichnet, dass** das Gas ununterbrochen während jeder Zeitspanne überwacht wird.

5. Verfahren nach einem der Ansprüche 2 bis 4, **dadurch gekennzeichnet, dass** die Schritte (a) und (b) zumindest einmal am Tag wiederholt werden.

6. Verfahren nach Anspruch 5, **dadurch gekennzeichnet, dass** die Schritte (a) und (b) bei einer relativ kalten oder langsamen Fermentierung einmal am Tag wiederholt werden.

7. Verfahren nach Anspruch 5, **dadurch gekennzeichnet, dass** die Schritte (a) und (b) bei einer relativ warmen oder starken Fermentierung zumindest einmal alle 8 Stunden wiederholt werden.

8. Verfahren nach einem der vorstehenden Ansprüche, **dadurch gekennzeichnet, dass** das Gas durch Aussetzen eines Indikatorstreifens, der selektiv auf das Verhandensein von H₂S anspricht zu dem Gas auf das Vorhandenssein von H₂S darin überwacht wird.

9. Verfahren nach Anspruch 8, **dadurch gekennzeichnet, dass** der Indikatorstreifen ein mit Zinkacetat imprägnierter Indikatorstreifen ist, und dass, nach dem Aussetzen zu dem Gas, der Indikatorstreifen mit einem ersten Reagenz umgesetzt wird, das eine Lösung aus 4-Amino-N,N-dimethylanilinsulfat (oder dessen Hydrochlorid) aufweist, und ein zweites Reagenz, das eine Lösung von Eisenchlorid aufweist, zugesetzt wird, wobei die Entwicklung einer blauen Farbe in der Mischung von Reagenzien das Verhandensein von H₂S in dem Gas feststellt.

## Revendications

1. Procédé pour la préparation d'une boisson alcoolisée par fermentation d'une liqueur fermentable, dans lequel une source d'azote assimilable est ajoutée à la liqueur pour accroître la teneur en azote assimilable de la liqueur, **caractérisé en ce que** le gaz se dégageant de la liqueur au cours de la fermentation est contrôlé en ce qui concerne la présence de H₂S dans ce gaz, et **en ce que** ladite source d'azote assimilable est ajoutée à la liqueur au cours de la fermentation, en réponse à la détection de H₂S dans ledit gaz.

2. Procédé suivant la revendication 1, **caractérisé en ce qu'**il comprend les étapes consistant (a) à contrôler ledit gaz pour déterminer la présence de H₂S dans ce gaz, pendant une période prédéterminée, (b) puis à ajouter une partie de ladite source d'azote assimilable à la liqueur en réponse à la détection de H₂S dans ledit gaz au cours de cette période, et (c) à répéter les étapes (a) et (b).

3. Procédé suivant la revendication 2, **caractérisé en ce que** les étapes (a) et (b) sont répétées de manière continue, du début à la fin de la fermentation.

4. Procédé suivant la revendication 2 ou la revendication 3, **caractérisé en ce que** ledit gaz est contrôlé de manière continue au cours de chacune desdites périodes.

5. Procédé suivant l'une quelconque des revendications 2 à 4, **caractérisé en ce que** les étapes (a) et (b) sont répétées au moins une fois par jour.

6. Procédé suivant la revendication 5, **caractérisé en ce que** les étapes (a) et (b) sont répétées une fois par jour dans le cas d'une fermentation à température relativement basse ou fermentation relativement lente.

7. Procédé suivant la revendication 5, **caractérisé en ce que** les étapes (a) et (b) sont répétées au moins toutes les 8 heures dans le cas d'une fermentation à température relativement élevée ou fermentation relativement poussée.

8. Procédé suivant l'une quelconque des revendications précédentes, **caractérisé en ce que** ledit gaz est contrôlé en ce qui concerne la présence de H₂S dans ce gaz en exposant au gaz une bandelette indicatrice qui est sélectivement sensible à la présence de H₂S.

9. Procédé suivant la revendication 8, **caractérisé en ce que** la bandelette indicatrice est une bandelette indicatrice imprégnée d'acétate de zinc, et **en ce que**, après l'exposition audit gaz, la bandelette indicatrice est amenée à réagir avec un premier réactif comprenant une solution de sulfate de 4-amino-N,N-diméthylaniline (ou de son chlorhydrate), auquel est ajouté un second réactif comprenant une solution de chlorure ferrique, le développement d'une couleur bleue dans le mélange de réactifs établissant ainsi la présence de H₂S dans ledit gaz.
